(19) Europäisches Patentamt
European Patent Office
Office européen des brevets

(11) **EP 3 928 713 B1**

(12) **EUROPEAN PATENT SPECIFICATION**

(45) Date of publication and mention
of the grant of the patent:
**16.08.2023 Bulletin 2023/33**

(21) Application number: **19920995.8**

(22) Date of filing: **14.11.2019**

(51) International Patent Classification (IPC):
**A61B 10/00** *(2006.01)* **A61B 3/113** *(2006.01)*

(52) Cooperative Patent Classification (CPC):
**A61B 5/0077; A61B 3/113; A61B 5/163;
A61B 5/168**

(86) International application number:
**PCT/JP2019/044653**

(87) International publication number:
**WO 2020/194849 (01.10.2020 Gazette 2020/40)**

(54) **EVALUATION DEVICE, EVALUATION METHOD, AND EVALUATION PROGRAM**

BEURTEILUNGSVORRICHTUNG, BEURTEILUNGSVERFAHREN UND
BEURTEILUNGSPROGRAMM

DISPOSITIF, MÉTHODE ET PROGRAMME D'ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.03.2019 JP 2019055142**

(43) Date of publication of application:
**29.12.2021 Bulletin 2021/52**

(73) Proprietor: **JVCKenwood Corporation
Yokohama-shi, Kanagawa 2210022 (JP)**

(72) Inventor: **HAKOSHIMA, Shuji
Yokohama-shi, Kanagawa 221-0022 (JP)**

(74) Representative: **Emde, Eric
Wagner & Geyer Partnerschaft mbB
Patent- und Rechtsanwälte
Gewürzmühlstrasse 5
80538 München (DE)**

(56) References cited:
WO-A1-2013/141098    WO-A1-2014/208761
WO-A1-2016/052646    WO-A1-2018/216347
US-A1- 2017 224 210    US-A1- 2020 069 230

## Description

### Field

The present invention relates to an evaluation apparatus, an evaluation method, and an evaluation program.

### Background

Recently, the number of people with developmental disabilities is said to be increasing. It has been known that the symptoms of developmental disabilities can be alleviated, and those with the disabilities can better adopt to their societies by finding the symptoms and starting education necessary for them at an early stage. Therefore, there has been a demand for an evaluation apparatus capable of evaluating people with the possibility of having developmental disabilities, objectively and efficiently.

Patent Literature 1 describes a technique for evaluating the possibility of a subject having attention-deficit hyperactivity disorder (ADHD), which is one of the developmental disabilities. This technique includes displaying a first image at a center of a display and displaying a second image around the first image; instructing the subject to look at the first image; detecting a gaze point of the subject on the display; and evaluating the possibility of the subject having ADHD based on a length of a gazing time by which the gaze point has remained within a region corresponding to each of the images.

US 2017 224 210 A discloses an inattention measurement device, system, and method in which an index value indicating an inattention level of a subject is calculated based on information relating to a history of a gazing point of a subject when presented with a task of searching for a prescribed target.

WO 2018 216 347 A discloses an evaluating device which is provided with: an image data acquiring unit which acquires image data relating to an eyeball of a subject; a point of gaze detecting unit which detects position data relating to a point of gaze of the subject; a display control unit which performs a display operation causing a plurality of objects to be displayed on a display screen, and a hide operation causing the objects to be hidden with a prescribed timing after the display operation; a region setting unit which sets a plurality of corresponding regions of the display screen, corresponding to each object; a determining unit which, on the basis of the point of gaze position data, determines whether or not the point of gaze is present in each corresponding region during a hide period in which the hide operation is being performed; a calculating unit which, on the basis of determination data, obtains the region data indicating each corresponding region, from among the corresponding regions, in which the point of gaze has been detected during the hide period; and an evaluating unit which obtains evaluation data of the subject on the basis of the region data.

US 2020 069 230 A discloses an evaluation device which includes: an image-data acquiring unit to acquire image data of an eyeball of a subject; a gaze-point detecting unit to detect position data of a gaze point of the subject based on the image data; a display control unit to perform a display operation to display objects on a display screen, and a non-display operation to hide the objects in predetermined timing; a region setting unit to set corresponding regions respectively corresponding to the objects on the display screen; a determining unit to determine, based on the position data, whether the gaze point is present in the corresponding region in a non-display period; an arithmetic unit to calculate, based on determination data, region data indicating the corresponding region in which the gaze point is detected in the non-display period; and an evaluating unit to calculate evaluation data of the subject based on the region data.

WO 2014 208 761 A discloses a control device which displays a diagnostic image obtained by combining a natural image and a pattern image on a display unit. An output control unit displays a first diagnosis image on the display unit, and after displaying the first diagnostic image, displays, on the display unit, a second diagnostic image in which the portion of the natural image and the pattern image are more similar than in the first diagnostic image.

WO 2013 141 098 A discloses an Asperger's diagnossi assistance method.

### Summary

### Technical Problem

A subject with ADHD has a tendency to move his/her gaze point more frequently due to the hyperactivity or impulsiveness, for example. The technique described in JP 2016-171849 A is capable of evaluating a movement of the gaze point indirectly, by comparing the length of the gazing time by which the gaze point has remained on the image at which the subject is instructed to look, and that on another image. However, there has also been a demand for a capability for making evaluation directly, in a manner suitable for characteristics of the ADHD.

The present invention is made in consideration of the above, and an object of the present invention is to provide an evaluation apparatus, an evaluation method, and an evaluation program capable of making evaluations directly, in a manner suitable for the characteristics that are unique to the subjects with ADHD.

### Solution to Problem

In accordance with the present invention, an evaluation apparatus, an evaluation method, and an evaluation program as set forth in the appended claims is provided. In particular, according to one aspect, there is provided an evaluation apparatus comprising: a display; a gaze point detecting unit configured to detect a position of a gaze point of a subject; a display controller configured to display an evaluation image including a main target and multiple sub-targets, and instruction information for instructing the subject to gaze at on the main target on the display; an region setting unit configured to set determination regions corresponding to the main target and the multiple sub-target; a determination unit configured to determine whether the gaze point is positioned within the determination regions based on the detected position of the gaze point; an arithmetic unit configured to calculate a movement count by which the gaze point has moved from one of the determination regions to another based on a determination result of the determination unit; and an evaluation unit configured to acquire evaluation data of the subject based on the movement count.

According to one aspect, there is provided An evaluation method comprising: detecting a position of a gaze point of a subject; displaying an evaluation image including a main target and multiple sub-targets, and instruction information for instructing the subject to gaze at the main target on a display; setting determination regions corresponding to the main target and the multiple sub-targets; determining whether the gaze point is positioned within the determination regions based on the detected position of the gaze point; calculating a movement count by which the gaze point has moved from one of the determination regions to another based on a determination result; and acquiring evaluation data of the subject based on the movement count.

According to one aspect, there is provided an evaluation program causing a computer to execute: a process of detecting a position of a gaze point of a subject; a process of displaying an evaluation image including a main target and multiple sub-targets, and instruction information for instructing the subject to gaze at the main target on a display; a process of setting determination regions corresponding to the main target and the multiple sub-targets; a process of determining whether the gaze point is positioned within the determination regions based on the detected position of the gaze point; a process of calculating a movement count by which the gaze point has moved from one of the determination regions to another based on a determination result; and a process of acquiring evaluation data of the subject based on the movement count.

**Advantageous Effects of Invention**

According to the present invention, it is possible to make evaluations directly, in a manner suitable for the characteristics unique to the subjects with ADHD.

**Brief Description of Drawings**

FIG. 1 is a schematic illustrating example of an evaluation apparatus according to an embodiment;
FIG. 2 is a functional block diagram illustrating one example of the evaluation apparatus;
FIG. 3 is a schematic illustrating example of an evaluation image displayed in a display;
FIG. 4 is a schematic illustrating example how instruction information is displayed on the display;
FIG. 5 is a schematic illustrating example of determination regions set to the display;
FIG. 6 is a schematic illustrating example of a trajectory followed by a gaze point of a subject without ADHD;
FIG. 7 is a schematic illustrating example of a trajectory followed by a gaze point of a subject with ADHD; and
FIG. 8 is a flowchart illustrating example of an evaluation method according to the embodiment.

**Description of Embodiments**

An evaluation apparatus, an evaluation method, and an evaluation program according to embodiments of the present invention will now be explained with reference to some drawings. The embodiment is, however, not intended to limit the scope of the present invention in any way. Elements in the embodiments described below include those that are replaceable by those skilled in the art, or those that are substantially the same.

In the explanation below, a three-dimensional global coordinate system will be established to explain positional relations among the parts. A direction in parallel with a first axis on a predetermined plane will be referred to as an X axis direction, and a direction in parallel with a second axis orthogonal to the first axis on the predetermined plane will be referred to as a Y axis direction. A direction in parallel with a third axis orthogonal to both of the first axis and the second axis will be referred to as a Z axis direction. An example of the predetermined plane includes an XY plane.

Evaluation Apparatus

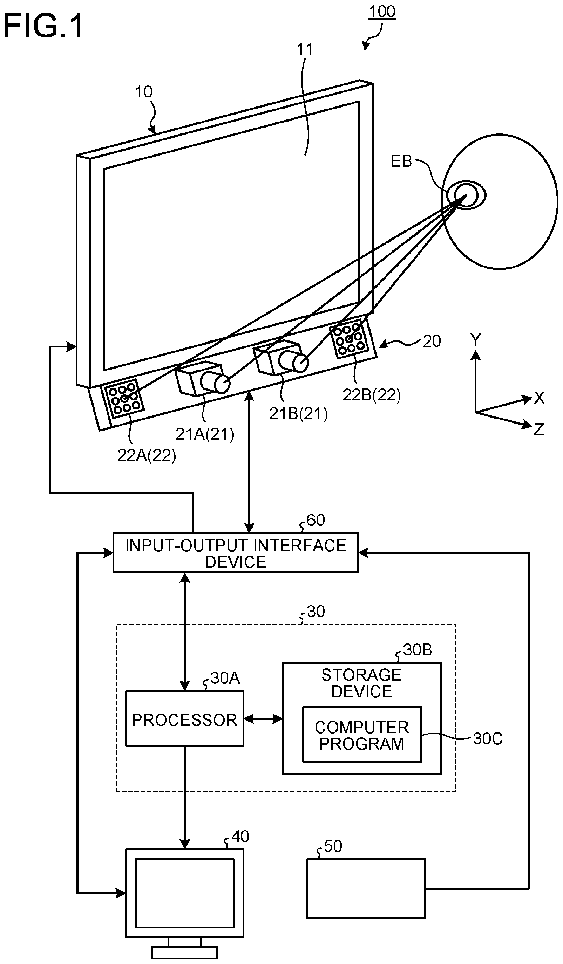

FIG. 1 is a schematic illustrating example of an evaluation apparatus 100 according to the embodiment. The evaluation apparatus 100 according to the embodiment detects a line of sight of a subject, and makes an evaluation related to attention-deficit hyperactivity disorder (ADHD) using the detection result. The evaluation apparatus 100 can detect the line of sight of the subject using various techniques, such as a technique for detecting the line of sight based on positions of pupils of the subject and positions of the images reflected on his/her corneas, and a technique for detecting the line of sight based on the positions of inner corners of the eyes of the subject and the positions of his/her irises.

As illustrated in FIG. 1, the evaluation apparatus 100 includes a display device 10, an image acquisition device 20, a computer system 30, an output device 40, an input device 50, and an input-output interface device 60. The display device 10, the image acquisition device 20, the computer system 30, the output device 40, and the input device 50 perform data communication via the input-output interface device 60. Each of the display device 10 and the image acquisition device 20 has a driving circuit not illustrated.

The display device 10 includes a flat panel display such as a liquid crystal display (LCD) or an organic electroluminescence display (OLED). In this embodiment, the display device 10 has a display 11. The display 11 displays information such as an image. The display 11 is substantially in parallel with the XY plane. The X axis direction is a left-and-right direction of the display 11, and the Y axis direction is an up-and-down direction of the display 11. The Z axis direction is a depth direction orthogonal to the display 11. The display device 10 may be a head-mounted display device. When the display device 10 is a head-mounted display device, a structure such as the image acquisition device 20 is disposed inside a head-mounted module.

The image acquisition device 20 acquires image data of the left and the right eyeballs EB of the subject, and transmits the acquired image data to the computer system 30. The image acquisition device 20 includes an image capturing device 21. The image capturing device 21 acquires the image data by capturing images of the left and the right eyeballs EB of the subject. The image capturing device 21 includes various types of cameras depending on the technique for detecting the line of sight of the subject. For example, when the technique for detecting the line of sight based on positions of the pupils of the subject and positions of the images reflected on his/her corneas is used, the image capturing device 21 includes infrared cameras, optical systems enabled to pass the near-infrared light having a wavelength of 850 [nm], for example, and an imaging device enabled to receive the near-infrared light. When the technique for detecting the line of sight based on positions of the inner corners of the eyes and positions of his/her irises of the subject is used, for example, the image capturing device 21 includes visible-light cameras. The image capturing device 21 outputs a frame synchronization signal. A cycle of the frame synchronization signals is 20 [msec], for example, but the embodiment is not limited thereto. The image capturing device 21 may also include a stereo camera having a first camera 21A and a second camera 21B, for example, but the embodiment is not limited thereto.

When the technique for detecting the line of sight based on the positions of the pupils of the subject and the positions of the images reflected on his/her corneas is used, for example, the image acquisition device 20 includes an illumination device 22 for illuminating the eyeballs EB of the subject. The illumination device 22 includes a light-emitting diode (LED) light source, and is capable of emitting near-infrared light having a wavelength of 850 [nm], for example. When the technique for detecting the line of sight based on the positions of the inner corners of the eyes of the subject and the positions of his/her irises is used, for example, the illumination device 22 does not necessarily need to be provided. The illumination device 22 emits a detection light in synchronization with the frame synchronization signal of the image capturing device 21. The illumination device 22 may also include a first light source 22A and a second light source 22B, for example, but the embodiment is not limited thereto.

The computer system 30 controls operations of the evaluation apparatus 100 comprehensively. The computer system 30 includes a processor 30A and a storage device 30B. The processor 30A includes a microprocessor such as a central processing unit (CPU). The storage device 30B includes a memory or a storage such as read-only memory (ROM) and a random access memory (RAM). The processor 30A executes an operation in accordance with a computer program 30C stored in the storage device 30B.

The output device 40 includes a display device such as a flat panel display. The output device 40 may also include a printer device. The input device 50 generates input data by being operated. The input device 50 includes a keyboard or a mouse for a computer system. The input device 50 may also include a touch sensor provided to a display of the output device 40 that is a display device.

In the evaluation apparatus 100 according to the embodiment, the display device 10 and the computer system 30 are separate devices. However, the display device 10 and the computer system 30 may be integrated with each other. For example, the evaluation apparatus 100 may include a tablet personal computer. In such a configuration, the tablet personal computer may be provided with the display device, the image acquisition device, the computer system, the input device, the output device, and the like.

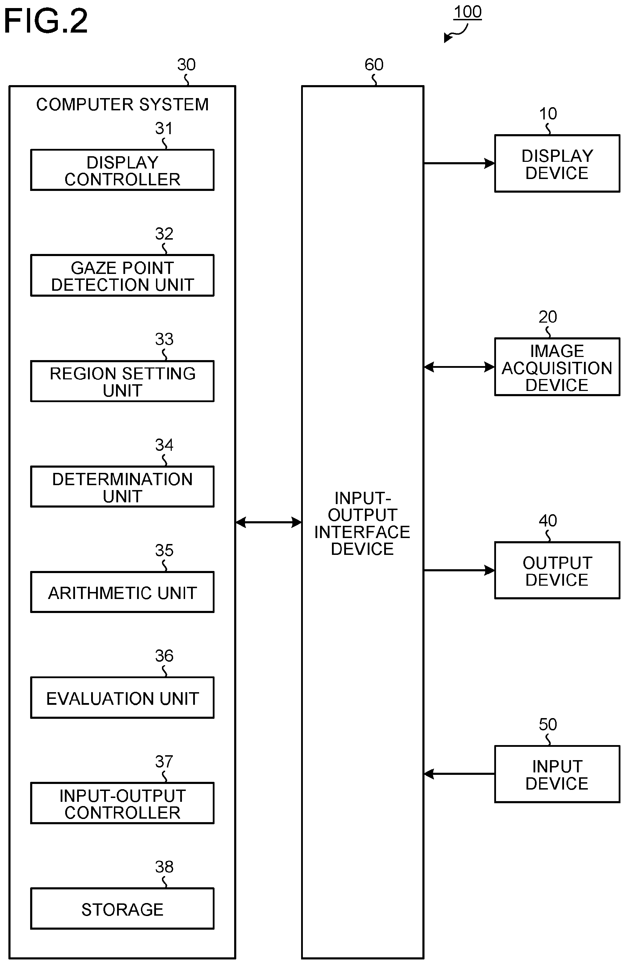

FIG. 2 is a functional block diagram illustrating one example of the evaluation apparatus 100. As illustrated in FIG. 2, the computer system 30 includes a display controller 31, a gaze point detection unit 32, a region setting unit 33, a determination unit 34, an arithmetic unit 35, an evaluation unit 36, an input-output controller 37, and a storage 38. The functions of the computer system 30 are exerted by the processor 30A and the storage device 30B (see FIG. 1). Some of the functions of the computer system 30 may be provided external to the evaluation apparatus 100.

The display controller 31 displays evaluation images on the display 11, after displaying instruction information, which will be described later, on the display 11. The display controller 31 may also display the instruction information and the evaluation images on the display 11 at the same time. In this embodiment, the evaluation image is an image including a main target and multiple sub-targets. In the evaluation image, the main target and the multiple sub-targets are included in the same image. The instruction information is information for instructing the subject to gaze at the main target in the evaluation image. The main target and the multiple sub-target are targets of the same type, for example. The "same type" herein means that these targets have the same characteristics and properties. Examples of the targets of the same type include the main target and the multiple sub-targets being persons, and the main target and the multiple sub-targets being animals other than humans, e.g., cats or dogs. The display controller 31 can display the evaluation image and the instruction information on the display 11 as an evaluation image, for example, but a display mode is not limited to the evaluation image, and may be a still image.

The gaze point detection unit 32 detects position data of the gaze point of the subject. In this embodiment, the gaze point detection unit 32 detects a vector of the line of sight of the subject, defined by the three-dimensional global coordinate system based on the image data of the right and left eyeballs EB of the subject, the image data being acquired by the image acquisition device 20. The gaze point detection unit 32 detects the position data of an intersection between the detected vector of the subject's line of sight and the display 11 of the display device 10, as the position data of the gaze point of the subject. In other words, in this embodiment, the position data of the gaze point is position data of the intersection between the vector of the subject's line of sight and the display 11 of the display device 10, defined by the three-dimensional global coordinate system. The gaze point detection unit 32 detects the position data of the gaze point of the subject at a specified sampling cycle. This sampling cycle may be set to a cycle of the frame synchronization signal output from the image capturing device 21, for example (e.g., 20 [msec]).

The region setting unit 33 sets determination regions corresponding to the main target and the multiple sub-targets on the display 11. In this embodiment, the determination regions set by the region setting unit 33 are not, in principle, displayed on the display 11. It is also possible for the determination regions to be displayed on the display 11 under control of the display controller 31, for example.

The determination unit 34 determines, for each of the determination regions, whether the gaze point is positioned within the determination region, based on the position data of the gaze point, and outputs a determination result as determination data. The determination unit 34 determines whether the gaze point is positioned within the determination regions at a specified determination cycle. As the determination cycle, for example, the cycle of the frame synchronization signal output from the image capturing device 21 (e.g., 20 [msec]) may be used. In other words, the determination cycle of the determination unit 34 is the same as the sampling cycle of the gaze point detection unit 32. Every time the gaze point detection unit 32 collects a sample of the position of the gaze point, the determination unit 34 makes a determination about the gaze point, and outputs the determination data. When the multiple determination regions are set, the determination unit 34 may determine, for each of the determination regions, whether the gaze point is positioned within the determination region, and output the determination data.

The arithmetic unit 35 calculates movement count data of the gaze point based on the determination data from the determination unit 34. The movement count data is data representing the number of times the gaze point has moved from one determination region to another. The arithmetic unit 35 checks the determination data every time the determination data is output from the determination unit 34 at the cycle described above, for example. If the region where the gaze point is determined to be positioned has changed from the previous determination result, the arithmetic unit 35 determines that the gaze point has moved between these regions. The arithmetic unit 35 has a counter for counting the number of times by which the gaze point is determined to have moved between the regions. When the gaze point is determined to have moved, the arithmetic unit 35 increments a value of the counter by one. The arithmetic unit 35 also includes a timer for detecting a time having elapsed from when the evaluation image is displayed on the display 11, and a management timer for managing a time for replaying the evaluation image.

The evaluation unit 36 acquires evaluation data of the subject based on the movement count data of the gaze point. The evaluation data includes data for evaluating whether the subject has exhibited an ability to keep his/her eyes on the main target and the multiple sub-targets displayed on the display 11.

The input-output controller 37 acquires data from at least one of the image acquisition device 20 and the input device 50 (e.g., the image data of the eyeballs EB or the input data). The input-output controller 37 also outputs data to at least one of the display device 10 and the output device 40. The input-output controller 37 may output a task assigned to the subject from the output device 40 such as a speaker.

The storage 38 stores therein the determination data, the movement count data, and the evaluation data. The storage 38 also stores therein an evaluation program for causing a computer to execute a process of detecting the position of the gaze point of the subject, a process of displaying the evaluation image including the main target and the multiple sub-targets and the instruction information for instructing the subject to gaze at the main target on the display 11, a process of setting the determination regions corresponding to the main target and the multiple sub-targets, a process of determining whether the gaze point is positioned within the determination region based on the detected position of the gaze point, a process of calculating the movement count data by which the gaze point has moved from one determination region to another based on the determination result, and a process of acquiring the evaluation data of the subject based on the movement count data.

Evaluation Method]

An evaluation method according to the embodiments will now be explained. In the evaluation method according to the embodiments, the possibility of ADHD of the subject is evaluated using the evaluation apparatus 100 described above.

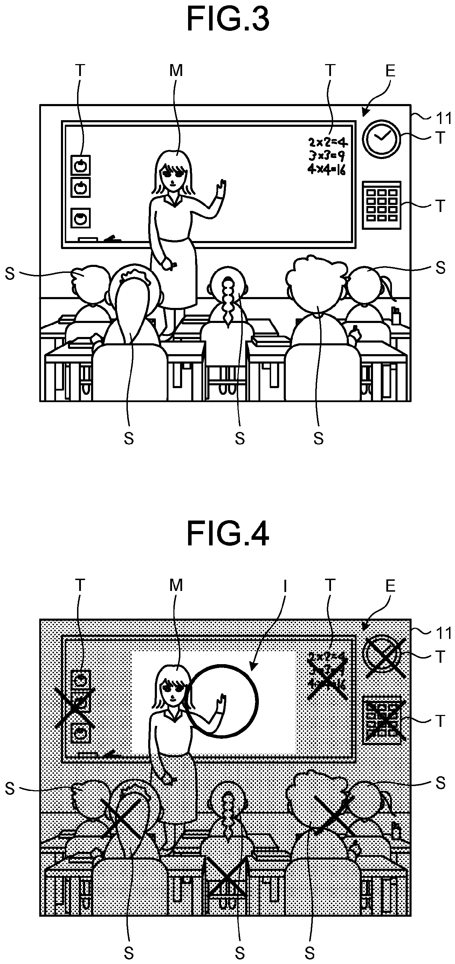

FIG. 3 is a schematic illustrating example of an evaluation image E displayed on the display 11. As illustrated in FIG. 3, the display controller 31 displays the evaluation image E including a main target M and multiple sub-targets S on the display 11. The main target M and the multiple sub-targets S are persons and the targets of the same type. In the example illustrated in FIG. 3, the teacher standing in front of a blackboard is the main target M. Students sitting on their chairs are the multiple sub-targets S. The evaluation image E includes other targets T such as a clock and a calendar in addition to the main target M and the multiple sub-targets S.

FIG. 4 is a schematic illustrating example of how instruction information is displayed on the display 11. As illustrated in FIG. 4, the display controller 31 displays, on the display 11, instruction information I for instructing the subject to gaze at the main target M in the evaluation image. In the example illustrated in FIG. 4, the display controller 31 displays regions other than the main target M at a lower luminance, and displays, as the instruction information I, an index represented as a circle at a portion corresponding to the main target M and indices represented crosses to portions corresponding to the multiple sub-targets S and the other targets T. The instruction information I is not limited to that using the evaluation image E. For example, the display controller 31 may also display instruction information such as characters on the display 11 without displaying the evaluation image E. The input-output controller 37 may also output a voice corresponding to the instruction information I, e.g., "Please fix your eyes on the teacher" from a speaker, in addition to the instruction information I being displayed. The input-output controller 37 may also output the voice corresponding to the instruction information I, e.g., "Please fix your eyes on the teacher" from a speaker, only as the voice, without displaying the instruction information I.

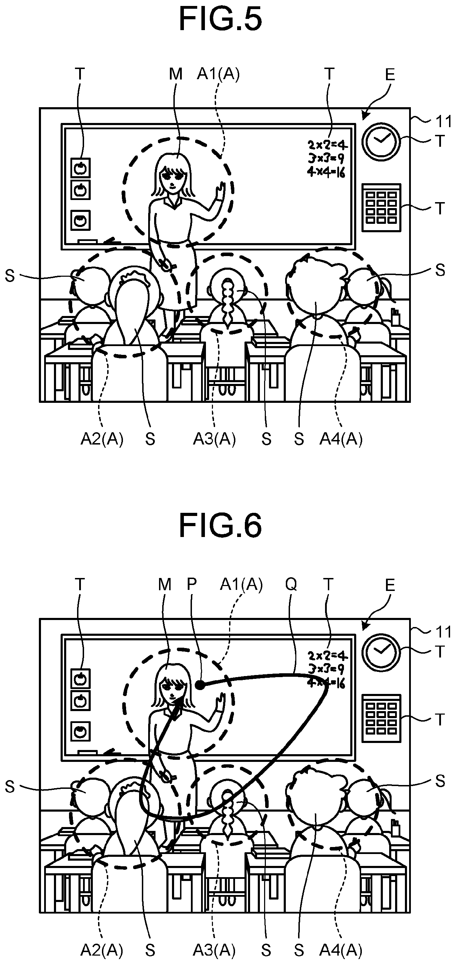

FIG. 5 is a schematic illustrating example of determination regions set to the display 11. As illustrated in FIG. 5, the region setting unit 33 sets determination regions A corresponding to the main target M and the multiple sub-targets S respectively. Hereinafter, when the determination regions A are to be distinguished from one another, the determination region corresponding to the main target M is sometimes referred to as a determination region A1, and the determination regions corresponding to the multiple sub-targets S are sometimes referred to as determination regions A2 to A4. The region setting unit 33 sets the determination regions A to regions not overlapping with one another. The determination regions A are not displayed on the display 11. The determination regions A1 to A4 are circular, for example, but are not limited to this shape, and may be another shape such as an oval or polygonal shape. Each of the determination regions A1 to A4 is set so as to include a part of the main target M or the sub-target S corresponding thereto, but may also be set to include the entire main target M or the sub-target S, without limitation thereto. Furthermore, each of the determination regions A2, A4 is set to cover the multiple sub-targets S, but the embodiment is not limited thereto, and one determination region may be set to each one of the multiple sub-targets S.

The characteristics of ADHD include, for example, carelessness, hyperactivity, and impulsiveness. A subject with ADHD has a tendency not to fully understand the instruction information I "Please fix your eyes on the teacher" due to their carelessness, or a tendency to move his/her gaze point frequently due to their hyperactivity or impulsiveness, for example. By contrast, a subject without ADHD has tendency to try to understand the instruction information I carefully, and to fix their eyes on the main target M. Therefore, in this embodiment, a subject is evaluated by instructing the subject to gaze at the main target M, detecting the number of times his/her gaze point has moved, and making the evaluation of the subject based on a detection result.

To begin with, the display controller 31 displays an evaluation image E on the display 11. The evaluation image E includes the main target M and the multiple sub-targets S of the same type, for example. After the evaluation image E is displayed, the display controller 31 displays, on the display 11, instruction information I for instructing the subject to gaze at the main target M included in the evaluation image E. The display controller 31 then stops displaying the instruction information I, and displays the evaluation image E. The region setting unit 33 sets the determination regions A (A1 to A4) to the main target M and the multiple sub-targets S respectively in the evaluation image E. The display controller 31 may also omit displaying the evaluation image E before displaying the instruction information I, and display the instruction information I from the start.

When displaying the evaluation image E, the display controller 31 may change a display mode in which the multiple sub-targets S are displayed. Examples of changing the display mode in which the multiple sub-targets S are displayed include displaying the sub-targets S with some actions that attract attentions of the subject, such as displaying the sub-targets S with moving their heads, or displaying their hair with moving. When the multiple sub-targets S are displayed in a different display mode, a subject with ADHD tends to be attracted more to the multiple sub-targets S, and to move his/her gaze point to the multiple sub-targets S impulsively. Therefore, by changing the display mode in which the multiple sub-targets S are displayed, a subject with ADHD can be evaluated highly accurately.

The gaze point detection unit 32 detects a position of the gaze point P of the subject at a specified sampling cycle (for example, 20 [msec]), during the period for which the evaluation image E is being displayed. When the position of the gaze point of the subject P is detected, the determination unit 34 determines whether the gaze point of the subject is positioned within the determination regions A1 to A4, and outputs determination data. Therefore, every time the position of the gaze point is sampled by the gaze point detection unit 32, the determination unit 34 outputs the determination data at a determination cycle that is the same as the sampling cycle.

The arithmetic unit 35 calculates movement count data indicating the number of times the gaze point P has moved during the period for which the evaluation image E is being displayed, based on the determination data. The movement count data is data indicating the number of times the gaze point has moved from one determination region A to another. The arithmetic unit 35 checks for the determination data every time the determination unit 34 outputs the determination data at the cycle explained above, and when the region having been determined to be where the gaze point is positioned has changed from the previous determination result, the gaze point is determined to have moved between the regions. When the gaze point is determined to have moved, the arithmetic unit 35 increments a counter for counting the movement count by one. For example, when the previous determination result is one indicating that the gaze point is positioned within the determination region A1, and the latest determination result is one indicating that the gaze point is positioned within one of the determination regions A2 to A4, the arithmetic unit 35 determines that the gaze point has moved between these regions, and increments the counter for counting the movement count by +1.

FIG. 6 is a schematic illustrating example of a trajectory Q of the gaze point P of a subject without ADHD. As illustrated in FIG. 6, a subject without ADHD has a tendency not to move the gaze point P largely because the subject has carefully understood the instruction information I. Even if there is some movement in the gaze point P, the movement remains to a degree circling about the main target M once or so, as illustrated in FIG. 6, for example. In the example of the trajectory Q illustrated in FIG. 6, the movement count by which the gaze point P has moved from one region to another is three.

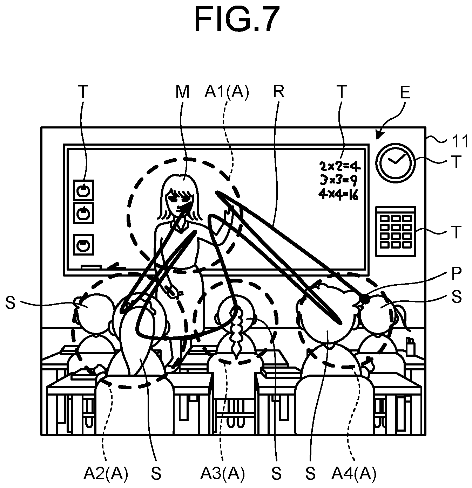

FIG. 7 is a schematic illustrating example of a trajectory R of the gaze point P of a subject with ADHD. As illustrated in FIG. 7, a subject with ADHD tends to move the gaze point P impulsively, and the gaze point P tends to move highly frequently. For example, as illustrated in FIG. 7, the subject has not gazed at the main target M from the beginning, and a detection is started at a timing when his/her eyes are fixed on the sub-target S. The subject then moves his/her gaze point P repeatedly between the main target M and the sub-targets S. In the exemplary trajectory R illustrated in FIG. 7, the movement count by which the gaze point P has moved from one region to another is eight.

The evaluation unit 36 then acquires an evaluation value based on the movement count data, and acquires evaluation data based on the evaluation value. In this embodiment, for example, denoting the movement count represented in the movement count data as n, an evaluation value ANS can be set as

$$ANS = n.$$

The evaluation unit 36 can acquire the evaluation data by determining whether the evaluation value ANS is equal to or more than a predetermined threshold K. For example, when the evaluation value ANS is equal to or more than the threshold K, the subject can be evaluated as being highly likely to have ADHD. When the evaluation value ANS is less than the threshold K, the subject can be evaluated as being less likely to have ADHD.

The evaluation unit 36 may also store the evaluation value ANS in the storage 38. For example, the evaluation unit 36 may store the evaluation values ANS of the same subject cumulatively, and makes an evaluation by comparing the evaluation value with that of a past evaluation. For example, when the evaluation value ANS is smaller than that of the past evaluation, it can be evaluated that the symptoms of the ADHD of the subject have alleviated compared with that of the previous evaluation. When the cumulative evaluation values ANS have gradually become smaller, it can be evaluated that the symptoms of the ADHD of the subject alleviating gradually.

In this embodiment, when the evaluation unit 36 outputs the evaluation data, the input-output controller 37 may output character data of the like, such as "The subject is less likely to have ADHD", or character data such as "The subject is highly likely to have ADHD", to the output device 40, depending on the evaluation data. When the evaluation value ANS is smaller than the past evaluation value ANS of the same subject, the input-output controller 37 may output character data or the like, such as "The symptoms of the ADHD have alleviated", to the output device 40.

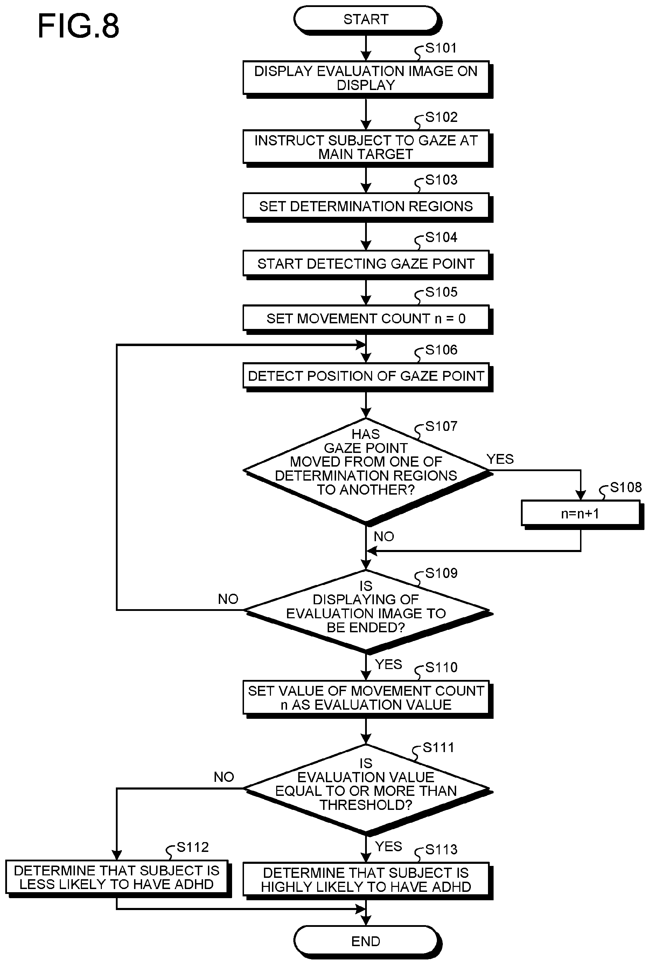

One example of the evaluation method according to the embodiment will now be explained with reference to FIG. 8. FIG. 8 is a flowchart illustrating one example of an evaluation method according to the embodiment. As illustrated in FIG. 8, in this evaluation method according to the embodiment, to begin with, the display controller 31 displays the evaluation image E on the display 11 (Step S101). The display controller 31 then displays the instruction information I for instructing a subject to gaze at on the main target M in the evaluation image E, on the display 11 (Step S102).

The display controller 31 then stops displaying the instruction information I, and displays the evaluation image E on the display 11. The region setting unit 33 then sets the determination regions A (A1 to A4) corresponding to the main target M and the multiple sub-targets S respectively in the evaluation image E (Step S103). The gaze point detection unit 32 then starts detecting the gaze point (Step S104). At this time, the arithmetic unit 35 resets the counter for counting the movement count (Step S105) .

The gaze point detection unit 32 collects a sample of the position of the gaze point at a predetermined sampling cycle, and detects the position of the gaze point (Step S106). Every time the gaze point detection unit 32 collects a sample of the position of the gaze point, the determination unit 34 determines whether the gaze point is positioned within the determination region based on the position of the gaze point, and outputs the determination data. Based on the determination data, the arithmetic unit 35 determines whether the gaze point has moved from one of the determination regions A1 to A4 to another (Step S107). When the gaze is determined to have moved (Yes at Step S107), the arithmetic unit 35 increments the counter for counting the movement count by +1 (Step S108), and executes Step S109 explained below.

When the Step S108 is executed, or when the gaze point is determined not to have moved from one of the determination regions A to another at Step S107 (No at Step S107), the arithmetic unit 35 determines whether the time for displaying the evaluation image E has ended (Step S109). When it is determined that the displaying time has not ended (No at Step S109), the processes at Step S106 and thereafter are repeated.

When it is determined that the displaying time has ended (Yes at Step S109), the evaluation unit 36 sets the value of the movement count as the evaluation value (ANS) (Step S110). The evaluation unit 36 then determines whether the evaluation value is equal to or more than the threshold (K) (Step Sill). When it is determined that the evaluation value is not equal to or more than the threshold (No at Step Sill), the evaluation unit 36 makes an evaluation that the subject is less likely to have ADHD (Step S112), and the process is ended. When the evaluation is equal to or more than the threshold (Yes at Step Sill), the evaluation unit 36 makes an evaluation that the subject is highly likely to have ADHD (Step S113), and the process is ended.

As described above, the evaluation apparatus 100 according to the embodiment includes the display 11; the gaze point detection unit 32 configured to detect the position of the gaze point of the subject; the display controller 31 configured to display the evaluation image E including the main target M and the multiple sub-targets S, and the instruction information I for instructing the subject to gaze at the main target M on the display 11; the region setting unit 33 configured to set determination regions corresponding to the main target M and the multiple sub-targets S respectively; the determination unit 34 configured to determine whether the gaze point is positioned within the determination regions A based on the position of the gaze point; the arithmetic unit 35 configured to calculate the movement count by which the gaze point has moved from one of the determination regions A to another based on the detected determination result; and the evaluation unit 36 configured to acquire the evaluation data of the subject based on the movement count.

The evaluation method according to the embodiment includes detecting the position of the gaze point of the subject; displaying the evaluation image E including the main target M and the multiple sub-targets S, and the instruction information I for instructing the subject to gaze at the main target M on the display 11; setting determination regions corresponding to the main target M and the multiple sub-targets S respectively; determining whether the gaze point is positioned within the determination regions A based on the position of the gaze point; calculating the movement count by which the gaze point has moved from one of the determination regions A to another based on the detected determination result; and acquiring the evaluation data of the subject based on the movement count.

The evaluation program according to the embodiment causes a computer to execute a process of detecting the position of the gaze point of a subject; a process of displaying the evaluation image E including the main target M and the multiple sub-targets S, and the instruction information I for instructing the subject to gaze at the main target M on the display 11; a process of setting determination regions corresponding to the main target M and the multiple sub-targets S respectively; a process of determining whether the gaze point is positioned within the determination regions A based on the position of the gaze point; a process of calculating a movement count by which the gaze point has moved from one of the determination regions A to another based on the detected determination result; and a process of acquiring the evaluation data of the subject based on the movement count.

A subjects with ADHD has a tendency not to fully understand the instruction information I due to their carelessness, for example, or a tendency to move their gaze point frequently due to their hyperactivity or impulsiveness, for example. Therefore, in this embodiment, after displaying the instruction information I for fixing his/her eyes on the main target M on the display 11, the determination regions A corresponding to the main target M and the multiple sub-targets are set, and the movement count by which the gaze point of the subject has moved between the determination regions A is counted. Hence, it is possible to make the evaluations directly in a manner suitable for the unique characteristics of the subjects with the ADHD.

In the evaluation apparatus 100 according to the embodiment, the display controller 31 displays the evaluation image E including the main target M and the multiple sub-targets S of the same type on the display 11. With this configuration, by using the targets of the same type as the main target M and the multiple sub-targets S, the multiple sub-targets S can attract the attention of the subject with ADHD efficiently, when the subject is asked to gaze at the main target M. Therefore, a highly accurate evaluation result can be acquired.

In the evaluation apparatus 100 according to the embodiment, when displaying the evaluation image E, the display controller 31 changes the display mode in which the multiple sub-targets S are displayed. With this configuration, by changing the display mode in which the multiple sub-targets S are displayed, the multiple sub-targets S can attract the attention of the subject with ADHD efficiently, when the subject is asked to gaze at the main target M. Therefore, a highly accurate evaluation result can be acquired.

The technical scope of the present invention is not limited to the embodiment described above. For example, explained in each of the embodiments is an example in which the evaluation apparatus 100 is used as an evaluation apparatus for evaluating the possibility of a ADHD of the subject, but the embodiment is not limited thereto. For example, the evaluation apparatus 100 may also be used as an evaluation apparatus for making an evaluation other than the possibility of a subject having ADHD, e.g., for evaluating the possibility of a subject having cognitive dysfunction and brain dysfunction, or evaluating a visual cognitive function of a subject.

Furthermore, explained in the embodiment described above is an example in which the timer is started at the timing at which the display controller 31 displays the evaluation image E, after displaying the instruction information I. However, the embodiment is not limited thereto. For example, it is also possible to start the timer and to start the evaluation at the timing at which the gaze point of the subject is confirmed to be positioned within the determination region A corresponding to the main target M, while the display controller 31 keeps on displaying the evaluation image E after displaying the instruction information I.

Furthermore, explained in the embodiment above is an example in which the determination regions A are set only to the main target M and the multiple sub-targets S, but the embodiment is not limited thereto. For example, it is also possible to set the determination regions to other targets T such as the clock or the calendar illustrated in FIGS. 3 and 5, for example, and use the determination regions in determining the movement count of the gaze point.

**Industrial Applicability**

The evaluation apparatus, the evaluation method, and the evaluation program according to the present invention may be used in a line-of-sight detecting apparatus, for example.

**Claims**

1. An evaluation apparatus comprising:

   a display (11);
   a detecting unit (32) configured to detect a position of a gaze point (P) of a subject;
   a display controller (31) configured to display an evaluation image (E) including a main target (M) and multiple sub-targets (S), and instruction information (I) which is an image for explicitly instructing the subject to gaze at the main target (M) on the display (11), and then to hide the instruction information (I);
   a region setting unit (33) configured to set determination regions (A, A1 to A4) corresponding to the main target (M) and the multiple sub-targets (S);
   a determination unit (33) configured to determine, after hiding the instruction information (I), whether the gaze point (P) is positioned within the determination regions (A, A1 to A4) based on the detected position of the gaze point (P);
   an arithmetic unit (35) configured to calculate a movement count by which the gaze point (P) has moved from one of the determination regions (A, A1 to A4) to another based on a determination result of the determination unit (33); and
   an evaluation unit configured to acquire evaluation data of the subject based on the movement count.

2. The evaluation apparatus according to claim 1, wherein the main target (M) and the multiple sub-targets (S) are of a same type.

3. The evaluation apparatus according to claim 1 or 2, wherein the display controller (31) further configured to display the multiple sub-targets (S) in a different display mode without changing a display mode of the main target (M) .

4. The evaluation apparatus according to any one of claims 1 to 3, wherein the display controller (31) further configured to display regions other than the main target (M) at a lower luminance.

5. An evaluation method comprising:

   detecting a position of a gaze point (P) of a subject;
   displaying an evaluation image (E) including a main target (M) and multiple sub-targets (S), and instruction information (I) which is an image for explicitly instructing the subject to gaze at the main target (M) on a display (11), and then to hide the instruction information (I) ;

setting determination regions (A, A1 to A4) corresponding to the main target (M) and the multiple sub-targets (S);

determining, after hiding the instruction information (I), whether the gaze point (P) is positioned within the determination regions (A, A1 to A4) based on the detected position of the gaze point (P);

calculating a movement count by which the gaze point (P) has moved from one of the determination regions (A, A1 to A4) to another based on a determination result; and

acquiring evaluation data of the subject based on the movement count.

6. An evaluation program causing a computer (30) to execute:

a process of detecting a position of a gaze point (P) of a subject;

a process of displaying an evaluation image (E) including a main target (M) and multiple sub-targets (S), and instruction information (I) which is an image for explicitly instructing the subject to gaze at the main target (M) on a display (11), and then to hide the instruction information (I);

a process of setting determination regions (A, A1 to A4) corresponding to the main target (M) and the multiple sub-targets (S);

a process of determining, after hiding the instruction information (I), whether the gaze point (P) is positioned within the determination regions (A, A1 to A4) based on the detected position of the gaze point (P);

a process of calculating a movement count by which the gaze point (P) has moved from one of the determination regions (A, A1 to A4) to another based on a determination result; and

a process of acquiring evaluation data of the subject based on the movement count.

**Patentansprüche**

1. Auswertungsvorrichtung, umfassend:

eine Anzeige (11);

eine Detektionseinheit (32), die konfiguriert ist zum Detektieren einer Position eines Blickpunkts (P) eines Subjekts;

eine Anzeigesteuerung (31), die konfiguriert ist zum Anzeigen eines Auswertungsbilds (E), das ein Hauptziel (M) und mehrere Teilziele (S) beinhaltet, und von Anweisungsinformationen (I), die ein Bild sind, um das Subjekt explizit anzuweisen, auf das Hauptziel (M) auf der Anzeige (11) zu blicken, und dann Verbergen der Anweisungsinformationen (I);

eine Bereichseinstelleinheit (33), die konfiguriert ist zum Einstellen von Bestimmungsbereichen (A, A1 bis A4) entsprechend dem Hauptziel (M) und den mehreren Teilzielen (S) ;

eine Bestimmungseinheit (33), die konfiguriert ist zum Bestimmen, nach dem Verbergen der Anweisungsinformationen (I), ob der Blickpunkt (P) innerhalb der Bestimmungsbereiche (A, A1 bis A4) positioniert ist, basierend auf der detektierten Position des Blickpunkts (P) ;

eine arithmetische Einheit (35), die konfiguriert ist zum Berechnen einer Anzahl von Bewegungen, mit welcher sich der Blickpunkt (P) von einem der Bestimmungsbereiche (A, A1 bis A4) zu einem anderen bewegt hat, basierend auf einem Bestimmungsergebnis der Bestimmungseinheit (33); und

eine Auswertungseinheit, die konfiguriert ist zum Erfassen von Auswertungsdaten des Subjekts basierend auf der Anzahl von Bewegungen.

2. Auswertungsvorrichtung nach Anspruch 1, wobei das Hauptziel (M) und die mehreren Teilziele (S) vom gleichen Typ sind.

3. Auswertungsvorrichtung nach Anspruch 1 oder 2, wobei die Anzeigesteuerung (31) ferner konfiguriert ist zum Anzeigen der mehreren Teilziele (S) in einem anderen Anzeigemodus, ohne einen Anzeigemodus des Hauptziels (M) zu ändern.

4. Auswertungsvorrichtung nach irgendeinem der Ansprüche 1 bis 3, wobei die Anzeigesteuerung (31) ferner konfiguriert ist zum Anzeigen von anderen Bereichen als dem Hauptziel (M) mit einer geringeren Luminanz.

5. Auswertungsverfahren, umfassend:

Detektieren einer Position eines Blickpunkts (P) eines Subjekts;

Anzeigen eines Auswertungsbilds (E), das ein Hauptziel (M) und mehrere Teilziele (S) beinhaltet, und von Anweisungsinformationen (I), die ein Bild sind, um das Subjekt explizit anzuweisen, auf das Hauptziel (M) auf einer Anzeige (11) zu blicken, und dann Verbergen der Anweisungsinformationen (I);

Einstellen von Bestimmungsbereichen (A, A1 bis A4) entsprechend dem Hauptziel (M) und den mehreren Teilzielen (S) ;

Bestimmen, nach dem Verbergen der Anweisungsinformationen (I), ob der Blickpunkt (P) innerhalb der Bestimmungsbereiche (A, A1 bis A4) positioniert ist, basierend auf der detektierten Position des Blickpunkts (P);

Berechnen einer Anzahl von Bewegungen, mit welcher sich der Blickpunkt (P) von einem der Bestimmungsbereiche (A, A1 bis A4) zu einem anderen bewegt hat, basierend auf einem Bestimmungsergebnis; und
Erfassen von Auswertungsdaten des Subjekts basierend auf der Anzahl von Bewegungen.

6.   Auswertungsprogramm, das einen Computer (30) veranlasst Folgendes auszuführen:

einen Prozess zum Detektieren einer Position eines Blickpunkts (P) eines Subjekts;
einen Prozess zum Anzeigen eines Auswertungsbilds (E), das ein Hauptziel (M) und mehrere Teilziele (S) beinhaltet, und von Anweisungsinformationen (I), die ein Bild sind, um das Subjekt explizit anzuweisen, auf das Hauptziel (M) auf einer Anzeige (11) zu blicken, und dann Verbergen der Anweisungsinformationen (I);
einen Prozess zum Einstellen von Bestimmungsbereichen (A, A1 bis A4) entsprechend dem Hauptziel (M) und den mehreren Teilzielen (S);
einen Prozess zum Bestimmen, nach dem Verbergen der Anweisungsinformationen (I), ob der Blickpunkt (P) innerhalb der Bestimmungsbereiche (A, A1 bis A4) positioniert ist, basierend auf der detektierten Position des Blickpunkts (P);
einen Prozess zum Berechnen einer Anzahl von Bewegungen, mit welcher sich der Blickpunkt (P) von einem der Bestimmungsbereiche (A, A1 bis A4) zu einem anderen bewegt hat, basierend auf einem Bestimmungsergebnis; und
einen Prozess zum Erfassen von Auswertungsdaten des Subjekts basierend auf der Anzahl von Bewegungen.

**Revendications**

1.   Appareil d'évaluation comprenant :

un dispositif d'affichage (11) ;
une unité de détection (32) configurée pour détecter une position d'un point de regard (P) d'un sujet ;
un dispositif de commande d'affichage (31) configuré pour afficher une image d'évaluation (E) comprenant une cible principale (M) et de multiples sous-cibles (S), et des informations d'instruction (I) qui sont une image pour ordonner explicitement au sujet de regarder la cible principale (M) sur le dispositif d'affichage (11), puis pour cacher les informations d'instruction (I) ;
une unité de réglage de région (33) configurée pour régler des régions de détermination (A, A1 à A4) correspondant à la cible principale (M) et aux multiples sous-cibles (S) ;

une unité de détermination (33) configurée pour déterminer, après avoir caché les informations d'instruction (I), si le point de regard (P) est positionné dans les régions de détermination (A, A1 à A4) sur la base de la position détectée du point de regard (P) ;
une unité arithmétique (35) configurée pour calculer un compte de mouvements par lequel le point de regard (P) s'est déplacé d'une des régions de détermination (A, A1 à A4) à une autre sur la base d'un résultat de détermination de l'unité de détermination (33) ; et
une unité d'évaluation configurée pour acquérir des données d'évaluation du sujet sur la base du compte de mouvements.

2.   Appareil d'évaluation selon la revendication 1, dans lequel la cible principale (M) et les multiples sous-cibles (S) sont d'un même type.

3.   Appareil d'évaluation selon la revendication 1 ou 2, dans lequel le dispositif de commande d'affichage (31) est en outre configuré pour afficher les multiples sous-cibles (S) dans un mode d'affichage différent sans changer un mode d'affichage de la cible principale (M).

4.   Appareil d'évaluation selon l'une quelconque des revendications 1 à 3, dans lequel le dispositif de commande d'affichage (31) est en outre configuré pour afficher des régions autres que la cible principale (M) à une luminance inférieure.

5.   Procédé d'évaluation comprenant :

détecter une position d'un point de regard (P) d'un sujet ;
afficher une image d'évaluation (E) comprenant une cible principale (M) et de multiples sous-cibles (S), et des informations d'instruction (I) qui sont une image pour ordonner explicitement au sujet de regarder la cible principale (M) sur un dispositif d'affichage (11), puis cacher les informations d'instruction (I) ;
régler des régions de détermination (A, A1 à A4) correspondant à la cible principale (M) et aux multiples sous-cibles (S) ;
déterminer, après avoir caché les informations d'instruction (I), si le point de regard (P) est positionné dans les régions de détermination (A, A1 à A4) sur la base de la position détectée du point de regard (P) ;
calculer un compte de mouvements par lequel le point de regard (P) s'est déplacé d'une des régions de détermination (A, A1 à A4) à une autre sur la base d'un résultat de détermination ; et
acquérir des données d'évaluation du sujet sur la base du compte de mouvements.

6. Programme d'évaluation amenant un ordinateur (30) à exécuter :

un processus pour détecter une position d'un point de regard (P) d'un sujet ;

un processus pour afficher une image d'évaluation (E) comprenant une cible principale (M) et de multiples sous-cibles (S), et des informations d'instruction (I) qui sont une image pour ordonner explicitement au sujet de regarder la cible principale (M) sur un dispositif d'affichage (11), puis cacher les informations d'instruction (I) ;

un processus pour régler des régions de détermination (A, A1 à A4) correspondant à la cible principale (M) et aux multiples sous-cibles (S) ;

un processus pour déterminer, après avoir caché les informations d'instruction (I), si le point de regard (P) est positionné dans les régions de détermination (A, A1 à A4) sur la base de la position détectée du point de regard (P) ;

un processus pour calculer un compte de mouvements par lequel le point de regard (P) s'est déplacé d'une des régions de détermination (A, A1 à A4) à une autre sur la base d'un résultat de détermination ; et

un processus pour acquérir des données d'évaluation du sujet sur la base du compte de mouvements.

# FIG.1

# FIG.2

100

## COMPUTER SYSTEM 30

### 31
DISPLAY CONTROLLER

### 32
GAZE POINT DETECTION UNIT

### 33
REGION SETTING UNIT

### 34
DETERMINATION UNIT

### 35
ARITHMETIC UNIT

### 36
EVALUATION UNIT

### 37
INPUT-OUTPUT CONTROLLER

### 38
STORAGE

## 60
INPUT-OUTPUT INTERFACE DEVICE

### 10
DISPLAY DEVICE

### 20
IMAGE ACQUISITION DEVICE

### 40
OUTPUT DEVICE

### 50
INPUT DEVICE

# FIG.3

# FIG.4

# FIG.5

# FIG.6

# FIG.7

# FIG.8

START

S101
DISPLAY EVALUATION IMAGE ON DISPLAY

S102
INSTRUCT SUBJECT TO GAZE AT MAIN TARGET

S103
SET DETERMINATION REGIONS

S104
START DETECTING GAZE POINT

S105
SET MOVEMENT COUNT n = 0

S106
DETECT POSITION OF GAZE POINT

S107
HAS GAZE POINT MOVED FROM ONE OF DETERMINATION REGIONS TO ANOTHER?

YES
S108
n=n+1

NO

S109
IS DISPLAYING OF EVALUATION IMAGE TO BE ENDED?

NO

YES
S110
SET VALUE OF MOVEMENT COUNT n AS EVALUATION VALUE

S111
IS EVALUATION VALUE EQUAL TO OR MORE THAN THRESHOLD?

NO
S112
DETERMINE THAT SUBJECT IS LESS LIKELY TO HAVE ADHD

YES
S113
DETERMINE THAT SUBJECT IS HIGHLY LIKELY TO HAVE ADHD

END

18

**EP 3 928 713 B1**

**REFERENCES CITED IN THE DESCRIPTION**

**Patent documents cited in the description**

- US 2017224210 A
- WO 2018216347 A
- US 2020069230 A
- WO 2014208761 A
- WO 2013141098 A
- JP 2016171849 A